# EUROPEAN PATENT APPLICATION

(11) **EP 0 548 031 A1**
(43) Date of publication of application: **23.06.1993**
(21) Application number: 92830662.0
(22) Date of filing: 10.12.1992
(51) Int. Cl.: A61C 1/00

(54) **Multifunction mobile appliance for multiple surgical applications**

(30) Priority: 11.12.1991 IT BO910466; 17.04.1992 IT BO920144
(71) Applicant: Boni, Alberto, I-40100 Bologna (IT)
(72) Inventor: Boni, Alberto, I-40100 Bologna (IT)
(74) Representative: Coppi, Cecilia

(57) **Abstract**

Mobile appliance that constitutes a surgical unit for multiple and multifunction uses, housing within a rectangular box structure a set of circuits and elements serving at least 2 instrument outlets with a conventional connector to interchangeable surgical instruments, and a triple-function syringe outlet, connected to a compressed air generating device with two motor compressors operating in parallel, associated with an air suction and purifying system consisting of three-stage microfilters and an air, water and spray cooling system.

## Description

This invention refers to a multifunction mobile unit for multiple surgical applications, characterized by the fact that it is transportable, that all its controls are within direct reach of the operator and that it has an extremely high operating autonomy.

Mobile container kits for various surgical operations are known to exist, for example in plastic surgery, orthopaedics and dentistry. Generally these are appliances housed in rectangular case-like containers which cannot be connected to the water mains, they have a small tank and consequently a very limited operating autonomy; in addition, they have electrical operating instruments which makes sterilization more difficult (e.g. micromotor). They are designed for a single function in that they are provided with individual operating units which are not interchangeable and cannot be used simultaneously, and they also have a cutter cooling fluid tank of extremely limited capacity which severely restricts operating autonomy and calls for service requirements which entail interruptions in use.

The aim of this invention is to permit the construction of a mobile appliance which overcomes the above-mentioned problems connected with the known technique and which extends the range and the opportunities for application by means of a multifunction high autonomy system with reduced or no operational assistance.

Another purpose is that the appliance is fitted with an extremely compact compressed air system without tank and with a triple filtration system reaching approximately 0.01 micron and with surgical suction.

The advantages of the invention are derived from the following characteristics: three instrument outlets, entirely pneumatic operation of the instruments, cooling of the rotating instruments with air or an air and water or other solution spray, direct connection to the water mains and/or large volume tank containing the fluid for the cooling spray.

The terminals of the structure are fitted with two known connectors, preferably of the Borden type, with the possibility of simultaneous use of these two outlets (thereby allowing two operators to work simultaneously) and one "syringe" attachment with three functions: air, water and spray, which can be selected with buttons on the "syringe" itself.

The spray cooling can be selected independently on each outlet, even when the said outlets are used simultaneously.

The distribution of the fluids to the various users is regulated by solenoid valves operated by button-type switches programmed by the operator and activated by the foot pedal switch.

The appliance can function by connection either to the water mains or independently, using its own stainless tank with corresponding capacity and an operating autonomy of approximately 7 hours (in continuous use), by connection either to the compressed air line or to its own separate compressor, and by normal connection to the electrical mains or to an independent power generator.

The compressor system for surgical use produces cooled air for medical applications without oil and water, with an efficiency of 99.999% and solid particles to a size of 0.01 micron, associated with a suction system.

The pumping unit consists of two oil-type noiseless motor compressors fitted with motor protection devices that stop the unit automatically in the case of overcurrent or when the temperature of the motor compressors reaches high values (100-110°C).

The motor protection devices (thermal cutouts) automatically restore the contacts when the temperature returns to a normal value (60-70°C). The forced cooling system remains activated when the unit is stopped.

The collection of aspirated fluids in a separate container is achieved by a vacuum of approximately 200 mmHg and the suction, which is continuous whether the compressor is in operation or not, is activated by one of the two motor compressors.

Condensate discharge and safety devices complete the advantages of the mobile multifunction appliance.

Other aims and advantages will become apparent from the following description and from the attached figures which illustrate schematically one form of execution of the invention.

With reference to these tables:
- Fig. 1 is a front view of the appliance;
- Fig. 2 is a rear view of the said appliance;
- Fig. 3 is a schematic overhead plan of the main connections inside the appliance, referring principally to the water system;
- Fig. 4 is a schematic view as above, referring chiefly to the pneumatic system connections;
- Fig. 5 is a plan of the mobile multifunction appliance incorporating the filtration elements and two other solenoid valves to interrupt the cooling air flow.
- Fig. 6 is a pneumatic operation diagram.

The appliance is contained inside a rectangular box-like housing of flat covers, the corners of which, formed by light metal sections together with sectional angular elements and a perforated base plate, serve as a frame and support and protection surface for all the components in the hydraulic and electrical circuits and the water tank.

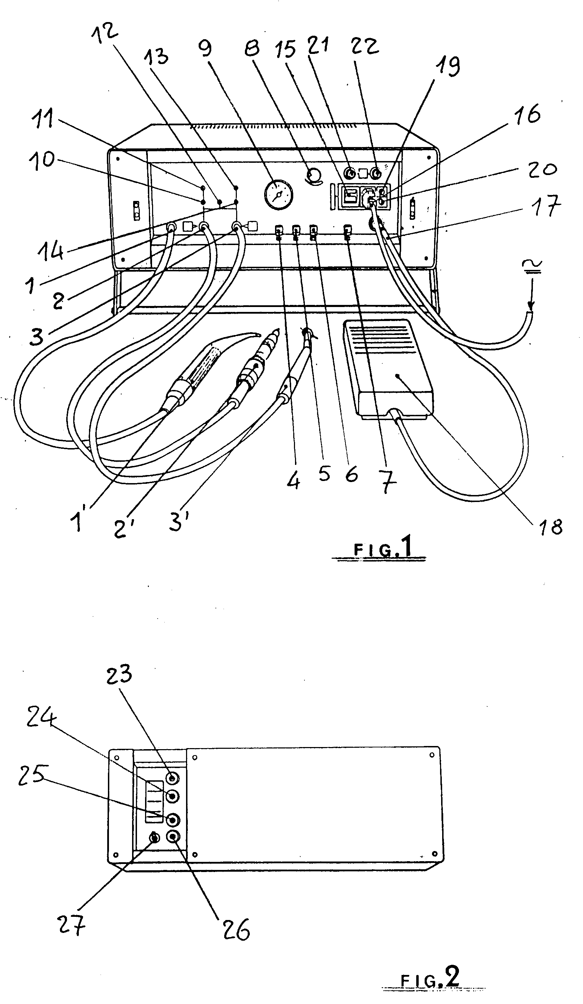

The front section illustrated in Fig. 1, within view of the operator, shows the fluid outlets marked 1, 2, 3 for the respective instruments 1', 2', 3' (the outlets 2 and 3 are preferably fitted with Borden connector).

The waterproof buttons for the solenoid valve selector switches are marked 4, 5, 6 and 7.

Pressing the button 4 activates the release of fluid for the cooling spray at outlet 2 and illuminates the indicator light 11; pressing the button 5 activates the outlet 3 or outlet 2 function and illuminates the indicator light for that outlet (outlet 2: light 10, outlet 3: light 14); pressing the button 6 activates the release of fluid for the spray at outlet 3 and illuminates the light 13; pressing the button 7 activates the simultaneous use of outlets 2 and 3 (the cooling spray selection remains independent for each outlet; even if the cooling spray is disconnected, the air cooling function still remains operative).

The safety light 16 carries the electrical current input, which is controlled by the fuses 19, 20, by means of the switch 15 which, through the transformer 30, carries the low voltage (approximately 12 V controlled by fuses 21, 22) to the solenoid valves for the programmed functions.

A foot pedal switch 18 is connected to and activates the appliance by means of an anti-rip plug 17.

The flow of water for the cooling spray is regulated by the rotating knob 8; the gauge 9 keeps the air pressure under visual control.

In the rear section (Fig. 2) the connector 23 is the fluid inlet (distilled water, physiological solution, disinfectant solutions) for conveyance to the tank 28 by means of the connector 23'; the connector 24 is the connection point for the compressed air that goes to the tank at point 24' and for the outlets 2, 3 and for the triple function syringe 1' and gauge 9; a unidirectional valve 37 prevents the backflow of the cooling fluid from the tank to the pneumatic circuit.

The connector 25, 25' marks the breather point for the air from the tank during filling, while the connector 26 marks the point of connection to the water mains.

By means of 26, 27' the mains water can be used to fill the tank 28 or to feed directly the cooling spray and the triple function syringe by operating the ON/OFF switch (valve) 27.

The water circuit (Fig. 2) also includes the tank 28, the pipe unions and the pipes, the flow regulator 8, and the solenoid valves 31, 32 for the release of water for the cooling spray water outlets 3 or 2 respectively.

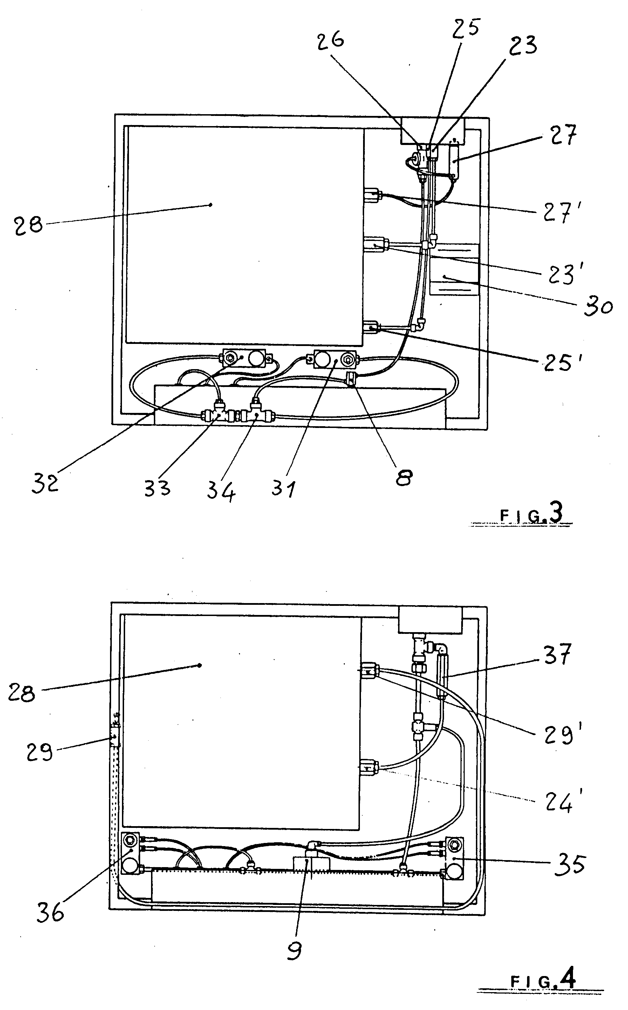

The pneumatic circuit (Fig. 4) includes the unidirectional valve 37 for filling the tank 28 with compressed air, a safety valve 29 from point 29' and two solenoid valves 35, 36 for the release of air of outlets 3 and 2 respectively (for the "rotor" and for cooling) in addition to the previously mentioned gauge 9 and the water outlet connectors 33, 34, the solenoid valves 32, 31 and the triple function syringe.

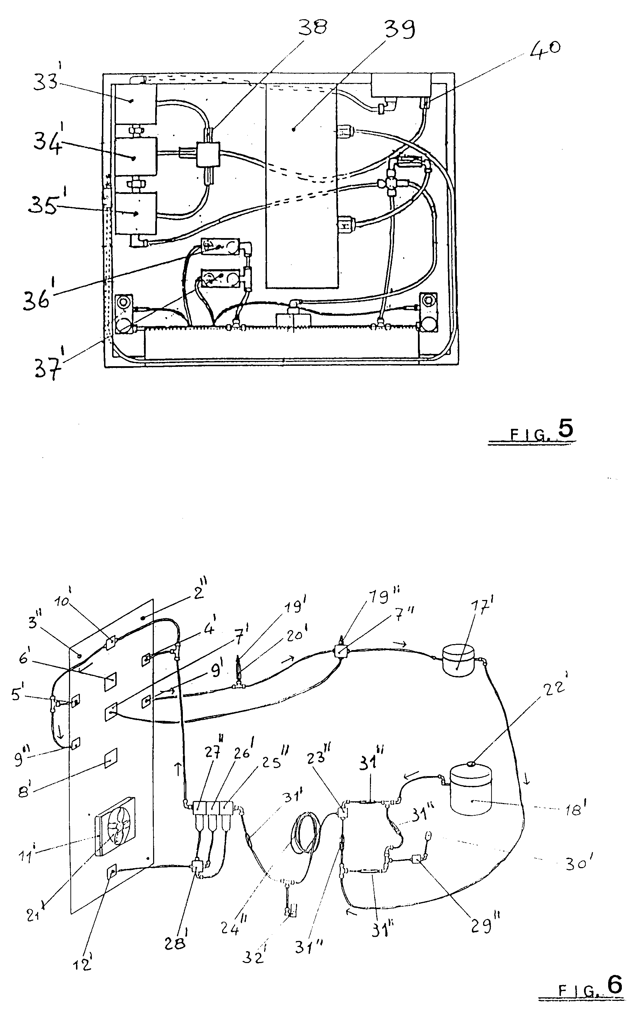

Fig. 5 shows how the circuit filtration elements can be installed in the multifunction appliance. In particular, together with a small tank 39 for medical solutions and with the condensate discharge 40, 3 filtration stages 33, 34 and 35 can be installed with unidirectional valves 38 and other solenoid valves 36 and 37 connected with the use envisaged for the variations of the multifunction appliance with just two instrument outlets. Thanks to this modification the appliance can also interrupt the tool's flow of cooling air by means of the two solenoid valves 36 and 37 controlled by two other buttons located on the console.

Thanks to this additional feature, three types of cooling are obtained:
a) continuous air cooling; b) continuous water (or other solution) cooling; c) continuous water and air spray cooling.

The overall pneumatic circuit diagram is shown schematically in Fig. 6.

The motor compressor 17' works under suction from one or the other circuit depending on whether it takes in air through the external organic fluid collection container from the operating area (inlet 9) or atmospheric air through the filter 19'' according to the position of the commutator 7' which operates the switch 7''.

Another function is that, when the vacuum pressure reaches a value of approximately 200 mmHg, the unidirectional valve 20' opens and part of the inducted air enters through the filter 19', while the vacuum of 200 mmHg still remains at the connector 9'.

The compressed air generated by the motor compressor 17' and the motor compressor 18', entering the former from the circuit and the latter through the filter 22', flows into in the mixer 23' from which, after travelling through the cooling coil 24' and the three stage filters 25', 26' and 27' up to 0.01 micron, which are cooled by the fan 21, reaches outlet 9' after regulation in 10 and arrives at the multifunction appliance.

The collected condensate of the filters flows into the unidirectional valves unit 28' and is discharged outside through the outlet 12'.

The unidirectional valves, indicated by 31'' in the various branches of the circuit, establish a pressure and flow balance between the two motor compressors 17' and 18'.

The optimal operating conditions are completed by other service elements such as the pressure switch 32', electrically connected to the solenoid valve 29' which, by means of the selector 30', discharges the air from inside the motor compressors and stops them when a pressure of 6 bars has been reached in the circuit.

A safety valve 31' against overpressure is also envisaged.

This invention achieves the stated aims and, specifically, it enables surgeons to work in peripheral structures without the need for specific fixed equipment.

Illustrated and described in schematic form and by way of example, this invention may be extended to all those accessory variants which, as such, fall within its scope.

Under actual implementation shapes, connections, regulations and materials may be of any kind whatsoever depending on requirements, and the technical details may be replaced by others that are technically equivalent, without thereby stepping beyond the scope of protection afforded by the following claims.

## Claims

1. MOBILE MULTIFUNCTION APPLIANCE FOR MULTIPLE SURGICAL APPLICATIONS, characterized by the fact that it is composed of a rectangular box-like housing containing a set of circuits and elements serving at least 2 instrument outlets with a conventional connector for surgical instruments which are interchangeable on the connectors of the said outlets, and one triple function syringe outlet, the said instruments being assisted by air cooling or cooling with a spray of air and water or other solution.

2. APPLIANCE, as described in claim 1, characterized by the fact that the operating controls, within direct reach of the operator, consist of waterproof push-buttons and switches and sterilizable button covers, a flow regulator, a pneumatic pressure control gauge and a foot pedal switch to activate the functions.

3. APPLIANCE, as described in claims 1 and 2 above, characterized by the fact that it is connected to the usual power mains or to a generator and by means of an incorporated transformer the voltage is reduced to the service values through the solenoid valves or other low voltage devices.

4. APPLIANCE, as described in the preceding claims, characterized by the fact that it is connected to the water mains and/or an incorporated tank which permits the use of medical solutions, and the liquid in the said tank can be discharged or recovered after use.

5. APPLIANCE, as described in the preceding claims, characterized by the fact that it has a pneumatic power pack for the surgical instruments with connection to a compressed air system or generator.

6. APPLIANCE, as described in claim 5, characterized by the fact that the said compressed air generator is provided with two motor compressors operating in parallel, associated with a suction system from the operating area, with microfilters for solid and liquid impurities, fitted with an hour meter, a safety valve against overpressure, a condensate discharge system to be activated at preset time intervals, and a compressed air and filter cooling system.

7. APPLIANCE, as described in the preceding claims, characterized by the fact that a commutator ensures that one of the motor compressors takes in air from the operating area for surgical aspiration or alternatively atmospheric air from the filter and introduces it into the compressed air circuit when necessary; otherwise it discharges the air freely into the atmosphere.

8. APPLIANCE, as described in the preceding claims, characterized by the fact that the said triple stage filtration microfilters deprive the air for medical use of oil, water and solid particles to a size of 0.01 micron, using the coalescent effect, with an efficiency of 99.999%.

9. APPLIANCE, as described in the preceding claims, characterized by the fact that the continuous suction system remains activated even if the compressed air is not used.

10. APPLIANCE, as described in the preceding claims, characterized by the fact that the organic fluids are sucked into a separate container by one of the motor compressors, with the possibility of using standardized collection containers.

11. APPLIANCE, as described in the preceding claims, characterized by the fact that the three-stage filtration system can also be housed in the mobile multifunction appliance.

12. APPLIANCE, as described in the preceding claims, characterized by the fact that a further two solenoid valves permit the said mobile multifunction appliance to achieve continuous air cooling, continuous water cooling, and continuous spray cooling.
